# EUROPEAN PATENT APPLICATION

(11) **EP 0 542 428 A1**
(43) Date of publication of application: **19.05.1993**
(21) Application number: 92309378.5
(22) Date of filing: 14.10.1992
(51) Int. Cl.: A61B 17/34

(54) **Trocar instrument**

(30) Priority: 14.10.1991 US 776496
(71) Applicant: DEXIDE, INC., Fort Worth Texas 76118-0995 (US)
(72) Inventor: Freitas, Michael W., North Richard Hills, Texas 76180 (US)
(74) Representative: Jackson, Peter Arthur

(57) **Abstract**

A surgical trocar (10) assembly is provided for insertion of a laparoscopic instrument through an inner abdominal wall. A sleeve is provided which can be expanded within the abdominal cavity to abut against the surface of the abdominal wall to hold the sleeve in place, in combination with insulation (28A) to electrically insulate the trocar assembly from the patient while the instrument is within the trocar assembly.

## Description

The present invention is directed to a medical device, and, more particularly, to a trocar assembly which can be inserted a short distance in the abdominal cavity during laparoscopic surgery and expanded to prevent the device from sliding in and out of a surgical incision.

Laparoscopic surgical procedures gain access to the interior of an anatomical cavity by first using an implement, such as a trocar spike, cannula or a needle having a sharpened point, to pierce or puncture the bodily tissues, muscles, membranes, or the like, which may form a portion of or surround the cavity wall.

Similarly, in many laparoscopic procedures, a small incision may be made in the skin of the patient along the abdomen, for example, and the sharp point of a larger penetrating implement, such as a trocar spike of suitable length and diameter, may be inserted into the incision, and pushed until the point punctures the cavity wall. A sleeve accompanies the implement into the puncture wound to serve as a lining for preserving the shape of the passageway created by the implement and for insertion of an endoscope, laparoscope, or the like, to view and operate upon organs within the cavity.

In many such applications, a trocar is used which incorporates a sleeve which may have a tendency to slide in and out of the incision in the abdominal wall, particularly when the surgeon is trying to move the laparoscopic instrument through the interior of the trocar sleeve into or out of the abdominal cavity.

In some endoscopic procedures, it has been found necessary to incorporate an electrosurgical procedure which uses a device which is electrically activated and incorporates blades, needles, and the like which are electrically activated by an electrical circuit which generates a radio frequency current with different wave form shapes to optimise tissue cutting, haemostasis, and the like. A hand piece is configured to receive a working electrode by which the radio frequency currents can be applied to the patient's tissue for cutting or haemostasis. Such electrosurgical devices have been utilised in laparoscopy in which the handpiece is carefully inserted through the trocar sleeve into the abdominal cavity. In such instances, when the handpiece is within the trocar sleeve, electric "arcing" can occur between the handpiece and the metallic components of the trocar sleeve causing electrical shocks to the patient and/or surgeon. Such metallic components include fixing elements penetrating the sleeve wall and used for securing parts of the sleeve together.

Typical of such electrosurgical apparatus is that as disclosed in US-A-4,754,754.

The present invention provides a surgical trocar assembly for insertion of a electrically actuated laparoscopic instrument through an inner abdominal wall. The trocar assembly comprises a trocar sleeve having a first end extending into the abdominal cavity through a laparoscopic incision in the abdominal wall, with the first end of the sleeve having a first external dimension for passage through the incision. Means are provided for expanding a portion of the first end of the sleeve within the abdomen so that the external dimension of the first end of the sleeve within the abdomen is expanded to a larger, second external dimension. The first end of the sleeve abuts the abdominal wall about the incision when expanded to the second external dimension to resist withdrawal of the trocar assembly from the abdominal cavity.

A passageway is defined that through the assembly and interior of the trocar sleeve for introduction and removal of the laparoscopic instrument relative to the abdomen. Insulation means are provided to electrically insulate the patient from the laparoscopic instrument while the instrument is within the assembly. The insulation means may comprise an insulation sheath which may be disposed within the sleeve and the passageway. Alternatively, the trocar sleeve may be formed of a conductive plastic having an electrical conductance of less than about 100 ohms per square inch.The sleeve may include an insulating elastomeric coating which is disposed thereon to electrically insulate the trocar assembly from the patient while the instrument is within the assembly.

In an alternative preferred embodiment, the trocar assembly is additionally provided with first and second interengageable gear members with the second gear member carried on the housing and the gear members being manually manipulatable to move the second end of a sleeve member between retracted and enlarged diameters.

The invention includes a laparoscopic surgical method in which an incision is made within the abdomen after which the trocar sleeve is inserted therein and manipulated to expand one end thereof to an enlarged diameter from a first, smaller diameter, such that, upon enlargement of the diameter of such end of the trocar sleeve within the abdomen, resistance is afforded to retractive movements of the trocar assembly relative to the inner abdominal wall.

After expansion of the end of the sleeve diameter to the enlarged position, the electrosurgical instrument is inserted through a passageway through the trocar assembly and the insulation provided between the exterior of the electrosurgical apparatus and the sleeve of the trocar assembly buffers the trocar assembly against "arcing" of electric current thereacross, thereby avoiding electric shock to the surgeon or the operator of the trocar assembly, or to the patient, possibly resulting in burning of tissue and the like during surgery, and/or establishing a field of static electricity immediate the area of surgery.

After use of the electrosurgical laparoscopic device inserted through the passageway within the trocar assembly, such device may be removed and the trocar sleeve subsequently manipulated, such that the diameter of the end of the sleeve within the abdominal wall is reduced from the expanded position to the retracted, initial position, and the trocar assembly removed from within the abdominal wall.
In the accompanying drawings:-

Fig. 1 is a cross-sectional view of a trocar sleeve forming a first embodiment of the invention prior to expansion.

Fig. 2 is a cross-sectional view of a trocar sleeve of Fig. 1 after expansion within the abdomen and interior of an abdominal wall.

Fig. 3 is a side view of a trocar used with a trocar sleeve.

Fig. 4 is a perspective view of a seal within the trocar sleeve.

Fig. 5 is an illustrative view of a first modification of the trocar sleeve where an expanding sponge is used.

Fig. 6 is an illustrative view of another modification of the trocar sleeve where an inflatable balloon is used.

Fig. 7 is a view in cross-section of a trocar sleeve forming a second embodiment of the present invention.

Fig. 8 is an exploded cross-sectional view of the trocar sleeve of Fig. 7.

Fig. 9. is an exploded perspective view of an alternative stop locking mechanism.

Fig. 10 is a cross-sectional view of another alternative stop locking mechanism.

Fig. 11 is a view in cross-section of a modified trocar sleeve.

Fig. 12 is a sectional schematic illustration of an alternative embodiment of the present invention in position prior to insertion into an abdominal cavity, with the cylindrical members being in normally retracted position relative to one another.

Fig. 13 is a view similar to that of Fig. 12, but illustrating the position of one of the cylindrical members relative to the other when the one cylindrical member's outer diameter is expanded, such as subsequent to insertion through the abdominal wall into the cavity, during surgery.

Fig. 14 is a perspective illustration of one of the cylindrical members and sleeve and an inter-engaging gear means.

Fig. 15 is a view similar to that of Fig. 14, illustrating the position of the respective components during activation such that the outer cylindrical sleeve is in expanded position.

Fig. 16 is an outer prospective view of the alternative embodiment of the present invention illustrating, in particular, the manual manipulating means through the housing and the seal assembly for insertion of an electrically activated laparoscopic instrument.

Fig. 17 is a view similar to that of Fig. 16, showing the insertion of the electrically activated laparoscopic instrument.

Fig. 18 is a view similar to Fig. 12 illustrating a device with insulation provided by an elastomeric coating.

Fig. 19 is a view similar to Fig. 13 showing the embodiment of Fig. 18 in expanded position.

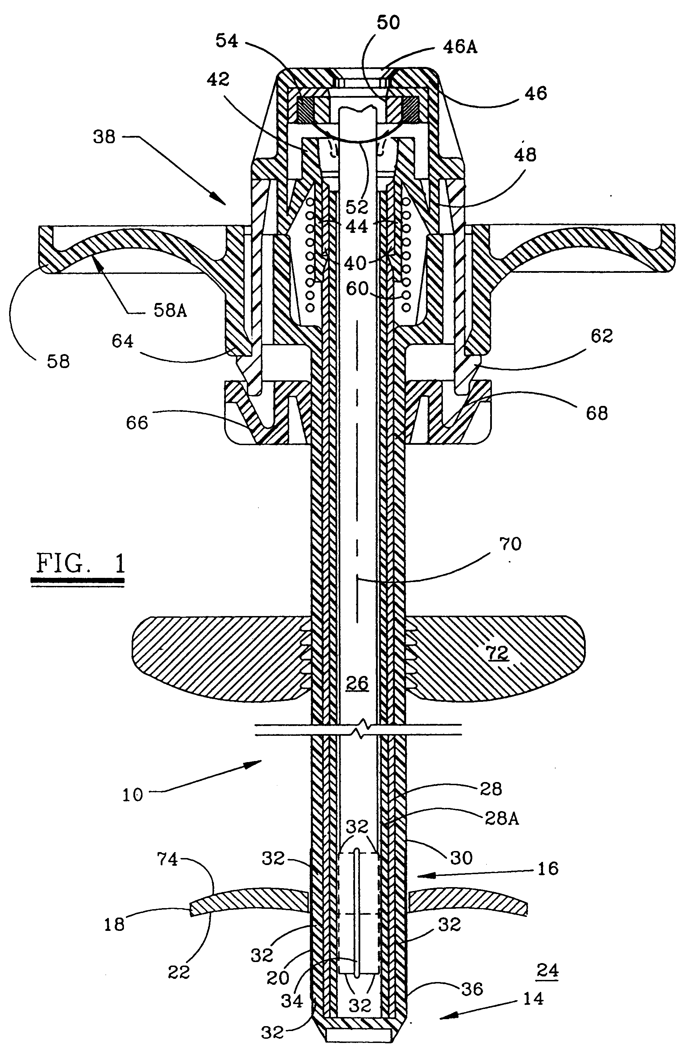

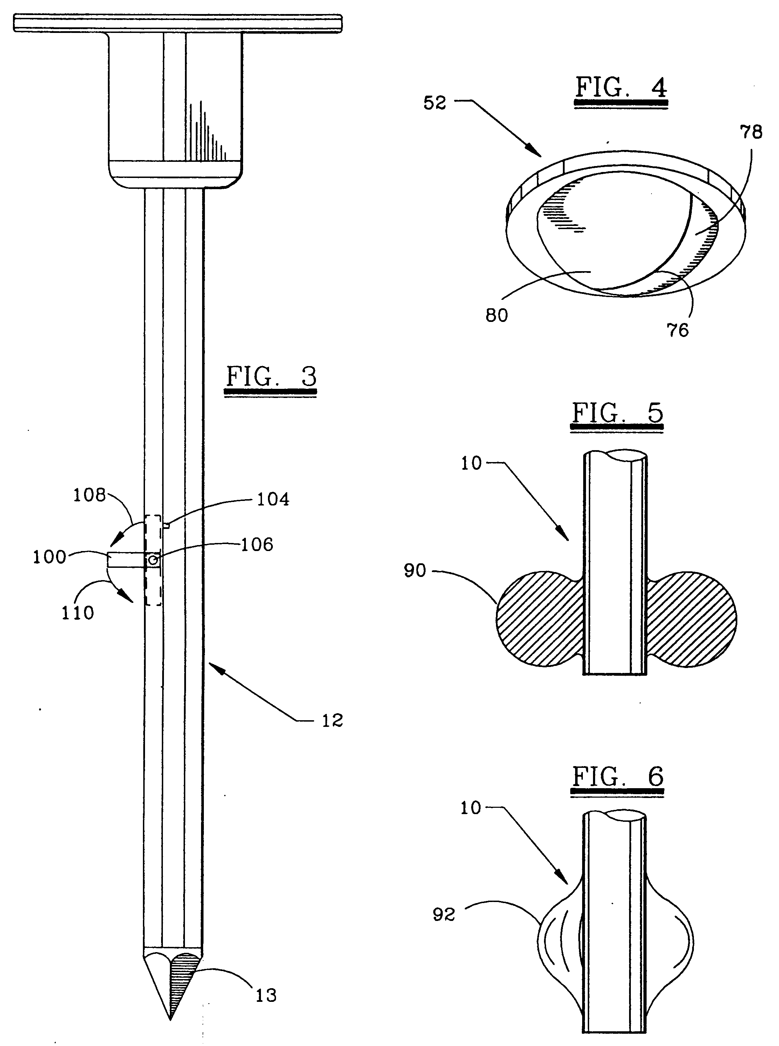

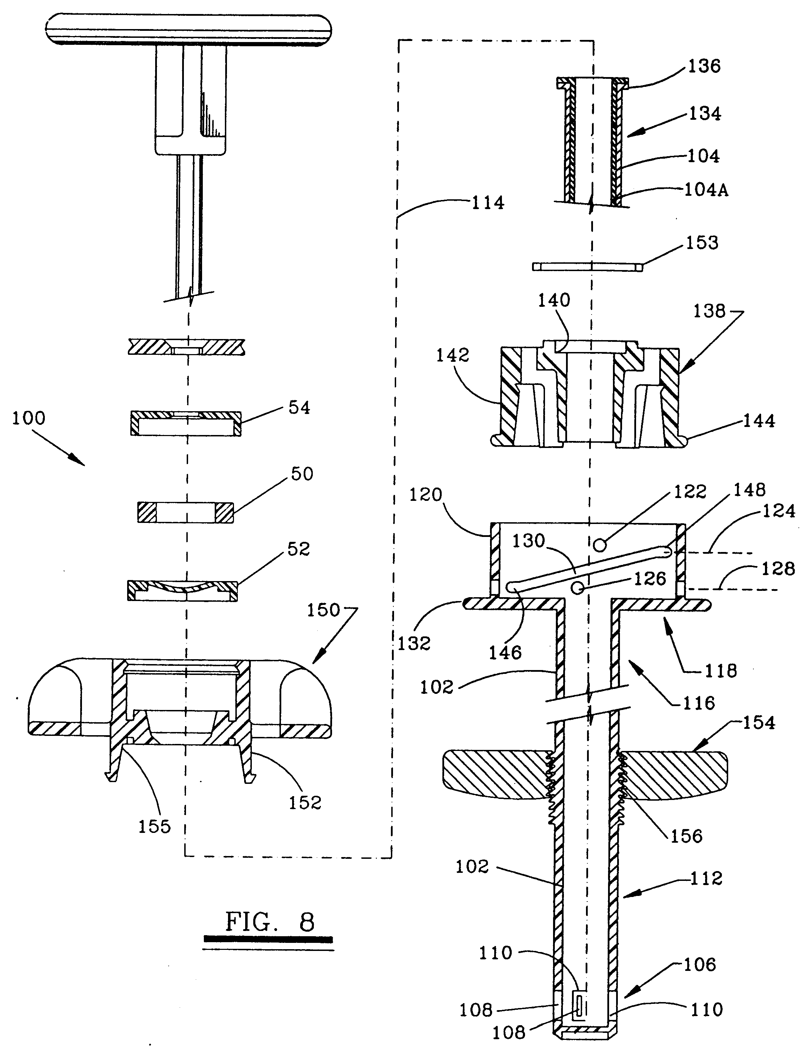

With reference now to FIGURES 1-3, a trocar sleeve 10 forming a first embodiment of the present invention is illustrated. With the assistance of a trocar 12, seen in FIGURE 3, a first end 14 of the sleeve 10 is inserted through an incision 16 in the abdominal wall 18 of a patient. The trocar 12 is inserted through passage 26 in the trocar sleeve so that only the sharp pointed end 13 extends from the first end 14 of sleeve 10. The pointed end 13 of trocar 12 then is pushed through the abdominal wall along with first end 14. The sleeve 10 is secured therein by expansion of a mushroom hinge 20 which abuts the inside surface 22 of the abdominal wall about the incision 16 to prevent premature withdrawal of the trocar sleeve. After removal of trocar 12, laparoscopic instruments and the like can be inserted into the abdominal cavity 24 through passage 26 in the trocar sleeve 10.

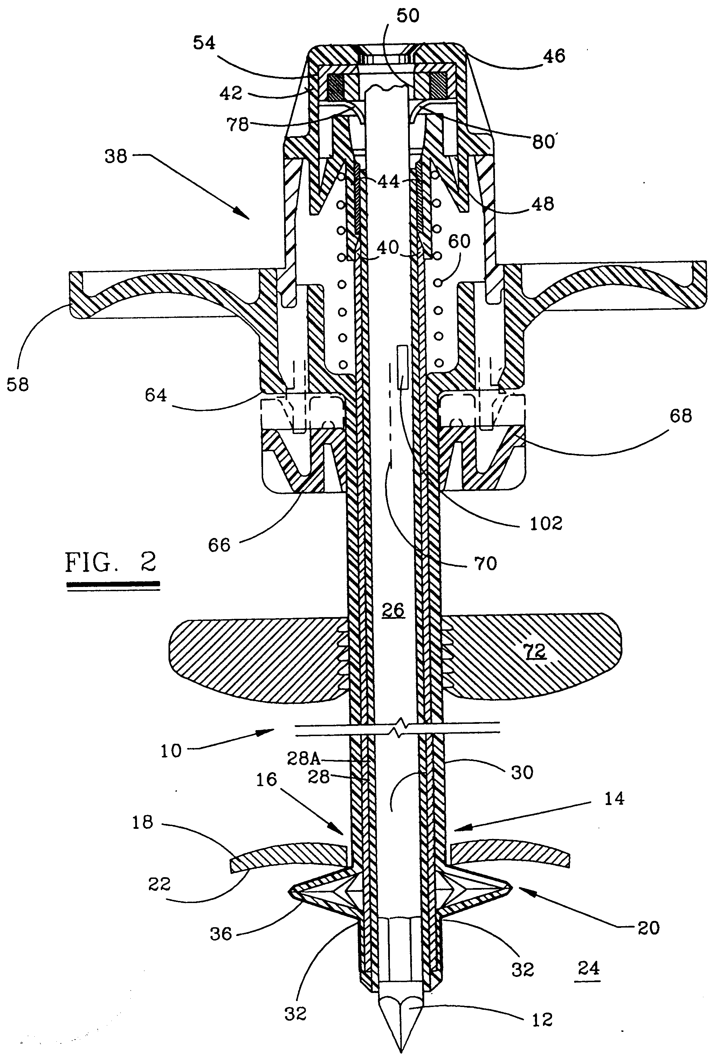

With reference to FIGURES 1 and 2, the trocar sleeve 10 can be seen to include an inner cylindrical metal sleeve 28 and a concentric outer cylindrical plastic sleeve 30 preferably of polypropylene or other suitable material. The sleeves 28 and 30 are secured indirectly one to another through an insulating sleeve member 28A at the first end 14. Hear the first end 14, the outer plastic sleeve 30 defines a mushroom hinge 20 with a series of hinge members 32 about the circumference of the sleeve 30 separated by elongated slits 34. An outer elastic coating or sleeve 36, preferably of latex, overlies the hinge member 32 and slits 34 to cover the mushroom hinge 20. Coating or sleeve 36 insures that no body tissue is trapped between portions of the mushroom hinge, particularly when contracting the hinge for removal of the sleeve 10 from the patient.

With continued reference to Figs. 1 and 2, interior of the metal sleeve 28 is disposed an additional concentric cylindrical sleeve member 28A extending all or part of the length of the member 28 to electrically insulate the trocar assembly from arcing caused by insertion therethrough of an electrically activated laparoscopic instrument while such instrument is within the trocar assembly. The inner sleeve 28A preferably is made of teflon or other chemical material known to those skilled in the art to insulate the electrically activated laparoscopic instrument from the metal tube 28. In lieu of providing a teflon sleeve 28A, any plastic or other material may be utilised to make the sleeve 28A provided that it is non-conductive.

Alternatively, the assembly as shown in Fig. 1 and Fig. 2, may provide insulation means in a form other than a sleeve 28A. For example, the outer sleeve 30 may be made of a conductive plastic with a conductance of less than about 100 ohms per square inch. In such configurations, such a plastic sheath will not act like a capacitor, thereby shielding the surgeon and patient against shock as a result of arcing attributable while the electrically activated laparoscopic instrument is within the trocar 10. The low conductivity of the sleeve effectively insulates the surgeon and patient from an electrical instrument inserted in the trocar while preventing the build up of charge on the trocar.

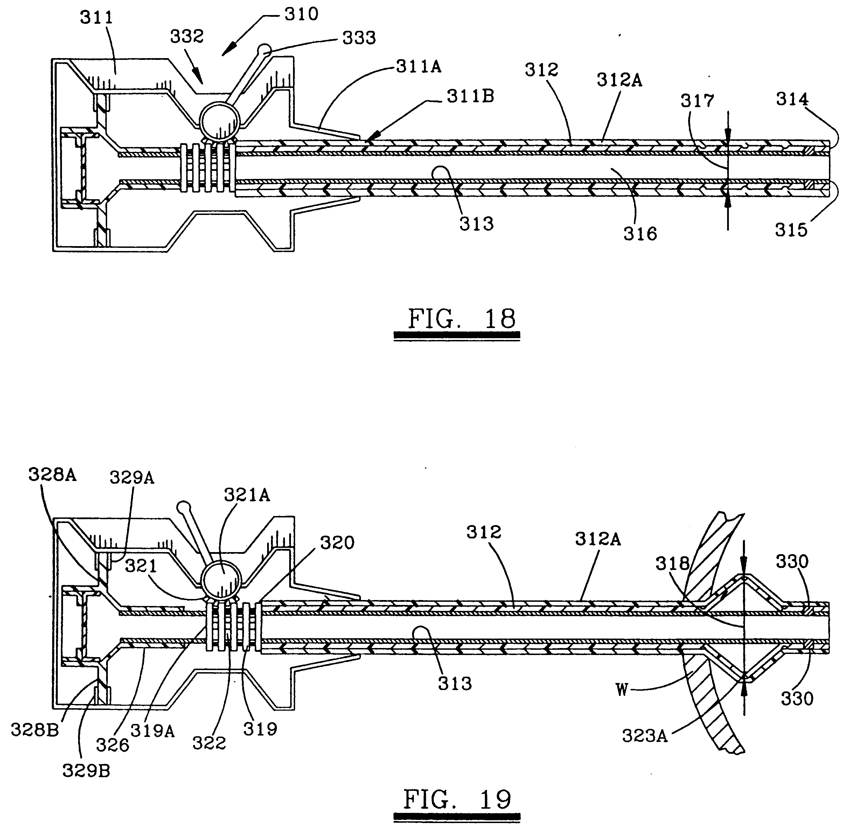

An additional alternative means of providing insulation means to the trocar assembly is by means of providing an elastomer sheath 312A over the outside of the plastic sheath 312, as shown in Figs.18 and 19. Such an elastomeric sheath may be made of silicone, latex of a material rubber.

At the end 38 of the trocar sleeve opposite the first end, the insulating sleeve 28A and sleeve 28 have a series of openings 40. A plastic end member 42 is secured to the sleeve 28, insulating sleeve 28A and outer sleeve 30 at end 38 by latches 44 received in the openings 40. A plastic seal retaining cap 46 is, in turn, secured to the end member 42 by latches 48 on the cap. On O.D. seal 50, a split seal 52, and a pack ring 54 are confined between the end member 42 and cap 46 to prevent the pressurized gas within the abdominal cavity from escaping through the passage 26 through the metal sleeve 28 as will be described hereinafter. Seals 50 and 52 are preferably of silicon rubber.

A handle 58 is secured to the outer plastic sleeve 30 at end 38. A coil spring 60 acts between the end member 42 and the handle 58 to urge the handle 58 toward the first end of the trocar sleeve.

In the configuration illustrated in FIGURE 1, the coil spring 60 is held in a compressed state between the end member 42 and the handle 58 as resilient latches 62 on the end member 42 are in contact with an end surface 64 of the handle.

A latch release 66 is received about the outer plastic sleeve 30 for movement relative the sleeve 30. The latch release 66 can be seen to have a conical camming surface 68 which can be moved into engagement with the ends of latches 62 to deflect the latches inward toward the axis 70 of the trocar sleeve 20. The latch release 66 can be activated by the surgeon directly, or, as an alternative, by providing a mechanism whereby withdrawal of the trocar after inserting the sleeve through incision 16 activates the latch release. The latches 62 will be deflected inward enough to release the handle 58 from the end member 42, which permits the spring 60 to expand to the position shown in FIGURE 2. As the spring expands, the spring forces the handle 58, and attached outer plastic sleeve 30, toward the first end relative to the end member 42 and inner metal sleeve 28 and insulating sleeve 28A. This causes the portions of the plastic sleeve 30 at the living hinges to bend and expand the mushroom hinge 20, as seen in FIGURE 2. It can be readily understood that the expanded mushroom hinge abuts the inside surface 22 of the abdominal wall 18 to resist removal of the trocar sleeve.

A dense rubber foam stop 72 can be frictionally engaged with the outer plastic sleeve 30. When the mushroom hinge has been expanded, the stop 72 can be slid downward along the plastic sleeve 30 toward the first end to contact the outer surface 74 of the abdominal wall 18 to resist movement of the trocar sleeve into the abdominal cavity.

It can be readily understood that the trocar sleeve 10 provides a stable platform for insertion of an electrosurgery or other laparoscopic instrument through the sleeve 10 into the abdominal cavity through passage 26 without concern for electric shocks. The expanded mushroom hinge 20 also forms a good seal with the patient's abdominal wall 18 to prevent CO₂ loss. Any tendency for the trocar sleeve 10 to move relative the abdominal wall as the instrument is being inserted or removed will be greatly reduced by the expanded mushroom hinge 20 and stop 72.

When the trocar sleeve 10 is to be removed from the patient, surface 46A is pressed towards the handle 58 at surface 58A in a manner similar to a syringe to collapse the mushroom hinge 20 and compress the spring 60 until the latches 62 again latch against surface 64 of the handle 58 to allow removal of the trocar sleeve. The mushroom hinge 20 can be collapsed and removed without having to move stop 72.

With reference now to FIGURES 1, 2 and 4, the mechanism for preventing gas from escaping the abdomen through the sleeve 10 will be described. When no electrosurgery or other laparoscopic instrument is inserted through the trocar sleeve 10, a split seal 52 prevents the escape of gas. The split seal 52 is formed of a resilient material which has a concave curvature facing the first end 14 of the trocar sleeve. A slit 76 is formed in the split seal which forms a first seal lip 78 and a second seal lip 80 on opposite sides of the slit 76. With the concave shape, the pressurized gas within the abdominal cavity acts to force the lips 78 and 80 together to form a tight seal to prevent the gas escape. When an instrument is inserted in end 38 of the trocar sleeve 10, the resilient lips 78 and 80 simply deflect away from the instrument, permitting the instrument to pass through the passage and into the abdominal cavity.

The O.D. seal 50 forms a seal against the outer cylindrical surface of an instrument as the instrument is inserted into the passage 26 of the trocar sleeve 10. With the combination of the O.D. seal 50 and split seal 52, very little gas is lost as instruments are inserted and removed from the trocar sleeve 10.

With reference to FIGURE 5, a first modification of the trocar sleeve 10 is illustrated. In the first modification, an expanding sponge 90 replaced the mushroom hinge 20 at the first end of the trocar sleeve 10. The sponge 90 can be expanded in a manner similar to the mushroom hinge 20 to hold the trocar sleeve within the abdominal cavity.

With reference to FIGURE 6, a second modification of trocar sleeve 10 is illustrated. In the second modification, an inflatable balloon 92 is mounted at the first end of the trocar sleeve which can be inflated to an expanded condition to secure the trocar sleeve within the abdominal cavity.

The sleeve 10 of the present invention reduces the frictional forces encountered by laparoscopic instruments being inserted into or removed from the sleeve as compared to prior designs. One reason for this advantage is that the entire sleeve 10 can be made shorter than past designs which required a longer length to insert through the abdominal wall to resist accidental removal of the sleeve from the patient. Another reason for the friction reduction is the use of efficient seals 50 and 52.

The trocar 12 can be used to automatically expand mushroom hinge 20 as the trocar is removed from sleeve 10. One possible mechanism for this is illustrated in FIGURES 2 and 3. A lever 100 can be pivoted to trocar 12 about hinge 106 and urged outwardly by a spring 60. The lever will be retracted into the trocar 12 as the trocar is inserted into the sleeve 10 to place the sleeve 10 through incision 16. When the trocar is removed from sleeve 10, the lever extends outward in the direction of arrow 108 through a slot 102 in the sleeve so that the lever 100 contacts latch release 66. Further movement of trocar 12 would cause the release 66 to move upward and deploy the mushroom hinge. The lever 100 can be mounted to retract back into trocar 12 in the direction of arrow 110 after sufficient force has been exerted on trocar 12 to move release 66 to allow the trocar to be removed from sleeve 10.

In one possible construction of a sleeve in accordance with the teachings of the present invention, the inner diameter of the sleeve could be about 5 to 6 mm. with the diameter of the expanded hinge about 0.845 inches. The stroke of the sleeve 30 to activate the hinge could be 3/8 inches and sleeve 36 could be 10 mils thick.

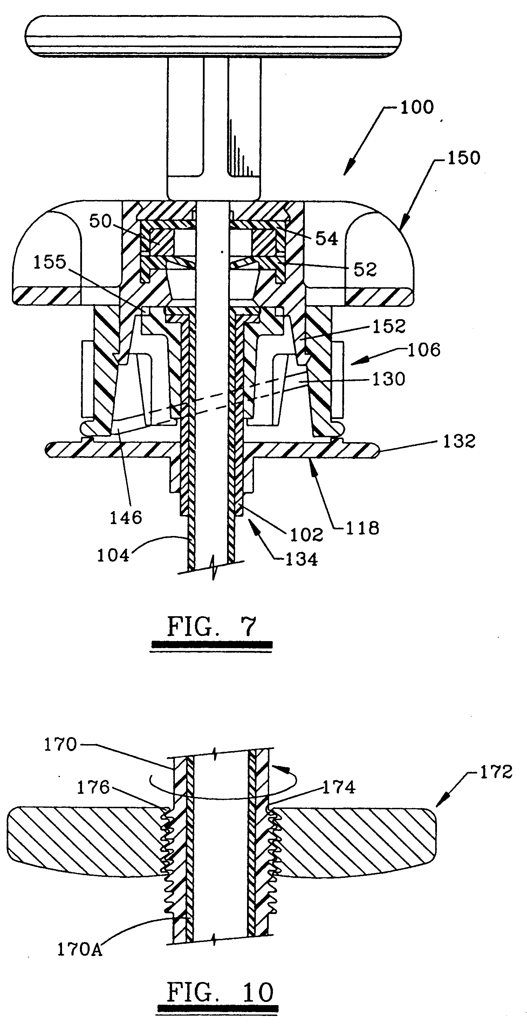

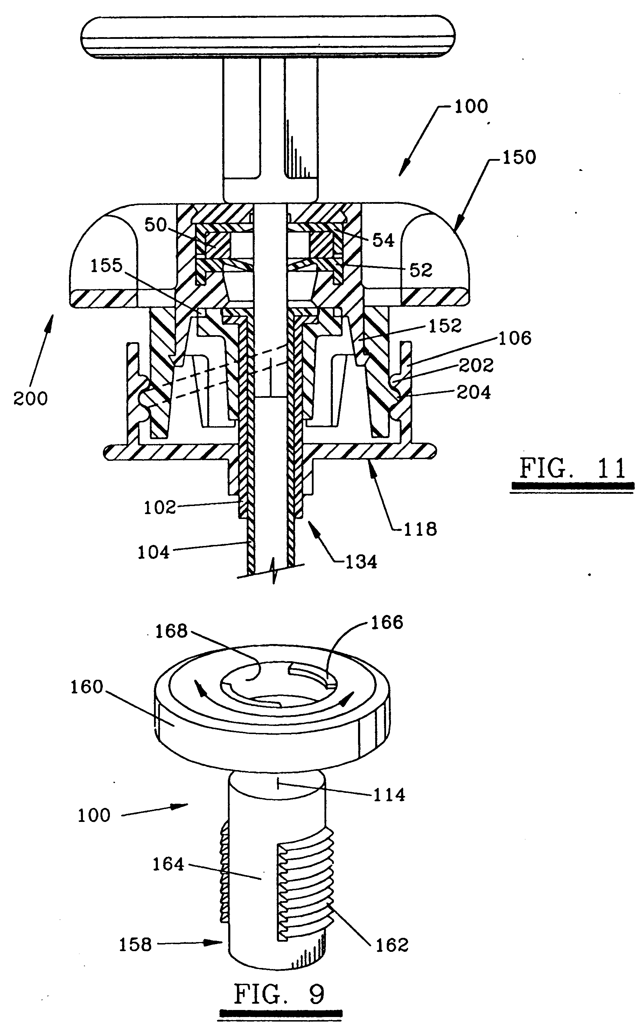

With reference now to FIGURES 7 and 8, a trocar 100 forming a second embodiment of the present invention is illustrated. The trocar 100 includes an outer cylindrical sleeve 102 and a concentric inner cylindrical electrically insulative sleeve 104. The sleeves 102 and 104 are secured together at a first end 106 of the trocar. The attachment is made by interlocking inwardly protruding lug 108 on sleeve 102 which fits within similarly shaped openings 110 in the inner cylindrical sleeve 104. The outer cylindrical sleeve 102 has formed thereon a mushroom hinge 112 much as hinge 20. As with trocar 12, relative movement between the outer cylindrical sleeve 102 and the inner cylindrical electrically insulative sleeve 104 will expand the mushroom hinge 112 to secure the trocar 100 in place, or to retract the mushroom hinge for insertion or removal of the trocar.

The end 116 of the outer cylindrical sleeve 102 opposite the first end 106 flares outwardly to form a cylindrical portion 118. The cylindrical portion 118 includes a cylindrical wall 120 with a series of apertures 122 through the wall in a first plane 124 perpendicular to the axis 114 and a similar series of apertures 126 in a second plane 128 perpendicular the first axis. A series of helically directed guide slots 130 are also formed in the wall 120 which extend from one end in the first plane to an opposite end in the second plane. The cylindrical portion 118 also has a radially extending flange 132 which assists a surgeon to hold the cylindrical sleeve.

The end 134 of inner cylindrical sleeve 104 is electrically insulated by sleeve 102. A member 138 is slid over the inner sleeve 104 so that the portion 136 of the inner cylindrical sleeve is received within an annular recess 140 in the member 138. The engagement between the portion 138 and recess 140 is sufficiently close to be essentially airtight, yet permit the member 138 to rotate about the axis 114 relative to the inner cylindrical sleeve 104. The member 138 also has a series of resilient arms 142 which extend into the cylindrical portion 118, with each arm 142 ending in a lug 144. Every other lug 144 is received in one of the guide slots 130. If the member 138 is rotated about axis 114 relative to the cylindrical portion 118, the interaction between the lugs and the guide slots 130 will cause the member 138, and sleeve 104, to move along axis 114. When the member 138 is turned so that lugs 144 abut a first end 146 of the guide slots, the mushroom hinge 112 is retracted and trocar can be inserted or removed from the patient. When the member 138 is rotated so that lugs 144 are guided along slots 130 to the second end 148 of the slots, the member 138 and inner cylindrical sleeve 104 are translated along the axis 114 to cause the mushroom hinge 112 to expand to lock the trocar within the patient. The intermediate lugs 144, i.e., those lugs 144 not received in one of the guide slots 130, act as detents in the retracted or extended positions, with the intermediate lugs 144 engaging apertures 126 when the mushroom hinge 112 is retracted and the lugs 144 engaging the apertures 126 when the mushroom hinge is deployed.

An end member 150 is snap fit to member 138 with a plurality of resilient arms 152. An O-ring 153 seals between end member 150 and member 138. The sealing effect of the O-ring is enhanced by a conical seal surface 155 on member 150 which urges the O-ring into compression between members 138 and 150 to form an effective seal. The end member 150 mounts the O.D. seal 54, split seal 52 and packing ring 50 as used in trocar 100.

A significant advantage of the trocar 100 is the deployment and retraction of the mushroom hinge by rotating one part of the trocar relative to the other about its elongated axis. Since the forces necessary to insert the trocar in the patient and remove the trocar from the patient are generally along the axis 114, there is little opportunity in trocar 100 for an inadvertent deployment or retraction of the mushroom hinge during insertion or removal of the trocar. Further, as the laparoscopic instruments are also inserted or removed by exerting forces along the axis 114, these procedures will likewise entail little risk of inadvertent actuation of the trocar.

The dense rubber foam stop 154 is seen to have ridges 156 in contact with the outer surface of the outer cylindrical sleeve 102. These ridges are oriented to make the movement of the stop along the sleeve 102 toward the patient relatively easy so that the stop can be quickly pushed against the outer surface of the patient's skin. However, the ridges resist movement along the sleeve in the opposite direction to prevent inadvertent loosening of the trocar 100 from the patient.

As shown in Fig. 8, insulation means may be provided by a teflon or other insulation-formed inner insulating member number 104A extending concentrically within the inner sleeve member 104, such that the inner sleeve may be made of metal. As with the embodiment shown in Figs. 1 and 2, such insulation means may also be provided in lieu of provision of sleeve 104A by means of a plastic sheath made of a non-conductive plastic for the outer sleeve 102 or by insulating the outer sleeve 102 with an elastomer sheath over the outside of the plastic of the outer sleeve 102.

With reference to FIGURE 9, the trocar 100 is shown with a modified outer sleeve 158 and stop 160. The outer sleeve 158 is formed with a discontinuous thread 162 which defines two unthreaded grooves 164 along axis 114. The stop 160 has a similar noncontinuous thread 166 with grooves 168. By turning the stop 160 to one orientation relative to the sleeve 158, the threads of one part are in the grooves of the other part, which permits the stop to be moved freely along axis 114 relative to the outer sleeve. When the stop is properly positioned relative to the patient, the stop need only be rotated 90 degrees to engage the threads of the stop and sleeve to lock the stop in the desired position.

With reference now to FIGURE 10, yet another modification of trocar 100 is illustrated which includes an outer cylindrical sleeve 170 and a stop 172 with electrically insulative sleeve member 170-A. The outer cylindrical sleeve 170 has a continuous thread 174 along its length. The stop has resilient rachet teeth 176 to engage the threads 174, which allows the stop to be easily moved in a direction along the axis 114 to move into engagement with the patient by the teeth 176 deflecting over the threads 174, but locks against threads 174 to resist removal. The threads 174 can be part of a continuous thread so that the stop can be backed off from the patient by rotating the stop to thread the stop back away from the patient.

FIGURE 11 illustrates a trocar 200 which is a modification of trocar 100. Trocar 200 uses mating threads 202 on cylindrical portion 118 and threads 204 on member 118 to move outer sleeve 102 and inner insulative sleeve 104 relative each other to activate the mushroom hinge. The threads have a pitch selected so that the desired axial movement between sleeves 102 and 104 can be achieved by rotating the threads less than one-half turn.

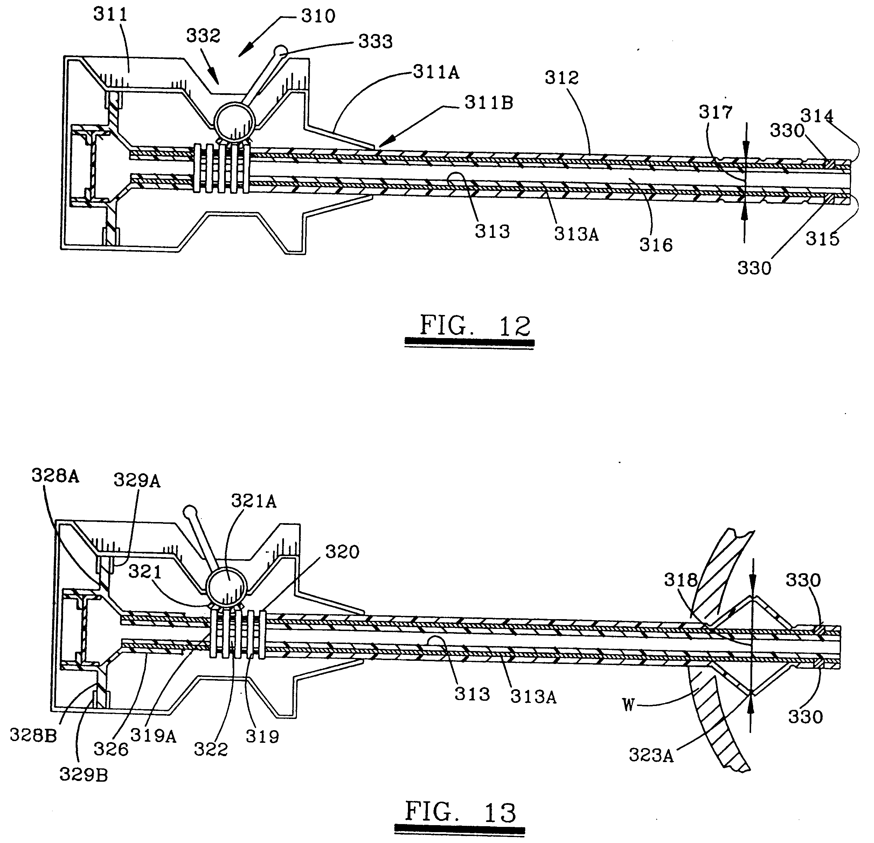

How with reference to Fig. 12, there is shown a trocar assembly 310 having a housing 311 with an upper opening 332 defined therein for receipt of the outwardmost end of a hand-manipulatable control assembly 333. The housing 310 has a distal end with an abutment skirt 311A thereon terminating in a receptacle 311B for receipt of first and second concentrically disposed cylindrical members 312 and 313 therethrough and into the housing 311. Placed between members 312 and 313 is an insulative sleeve 313A.

A passageway 316 extends completely though the assembly 310 and the innermost of the cylindrical members 313 to an opening 315. An end 314 is defined on the distal end of the first elongate cylindrical member 312, which is inserted into the abdominal cavity. As shown in Fig. 12, the first elongate cylindrical member 312 has a retracted outer diameter 317.

Now referring to Fig. 13, the first and second elongate cylindrical members 312 and 313 and the insulative sleeve 313A are secured, one to another by means of plastic securements 330 radially spaced there-across at the distal end.

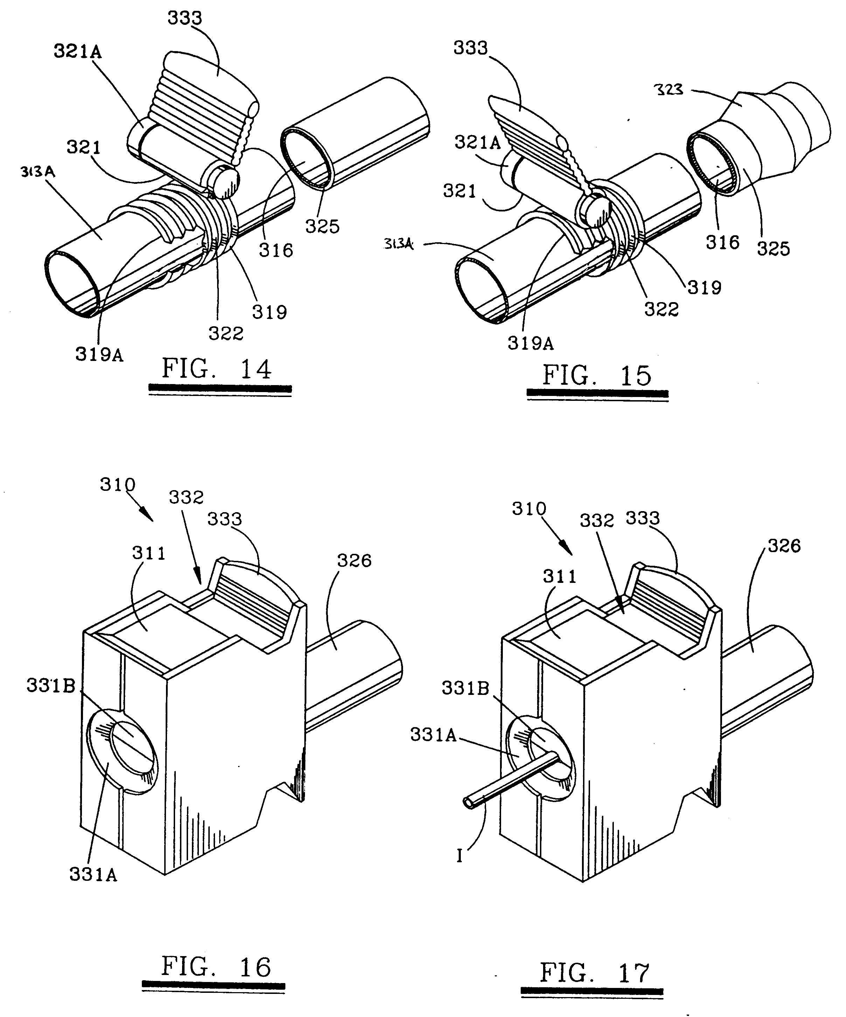

The first elongate cylindrical member 312 carries thereon a portion within the housing 311 defining a gear member 319 having inter-engaging teeth at 320 thereon and, preferably, in a circular spaced configuration, 322. A cylindrical extension 326 has a no-go shoulder 327 (Fig. 14) thereon which contacts the end 319A of the gear member 319 when the cylindrical members 312, 313A are moved to the normally retracted position, as shown in Fig. 12, to thereby limit travel of the moving cylindrical member in one direction, i.e., when the cylindrical members 312, 313A are moved by the control 333 from the expanded position, Fig. 13, to the retracted position, Fig. 12.

Companion inter-engaging teeth members 321 are provided circularly around a portion of a ring component 321A of the control 333 for inter-engagement with the teeth 320 on the gear members 319 of the first elongate cylindrical member 312.

The extension member 326 has a series of outwardly protruding wing members, 328A, 328B which are inserted in companion slots 29A, 329B on the housing 311 for securement thereto. An outer seal member 331A and companion lip seal 331B, as shown in Fig. 16, prevent transmission of gasses, and other fluids, from within the passageway 316, when the assembly 310 is within the abdominal cavity, during surgery.

An auxiliary instrument I, such as an electrically activated cutting device, may be inserted through the seal member 331B and into the passageway 316, subsequent to insertion of the assembly 310 within the abdominal cavity and manipulation of the first elongate cylindrical member 312 to the expanded outer diameter 318, as shown in Fig. 13.

How referring to Fig.'s 14 and 15, a sleeve portion 325 is defined immediate the distal end of the first elongate cylindrical member 312 and has a series of radially extending serrated flexing members 323 thereon with openings inter-defined there between. A serration 323A is cut on each of the flexing members 323 to permit flexing movement to the expanding outer diameter position 318.

As shown in Fig's. 12 and 13, the embodiment shown therein contains an insulating sleeve 313A made of teflon or other insulating material, the sleeve 313A being concentrically securingly disposed around the outer diameter of the inner sleeve member 313.

As with the other embodiments shown in the previous Figs., the embodiments shown in Figs. 12 and 13 may provide insulation means other than by positioning of a concentrically disposed inner teflon or other insulated sleeve member 313A, by means of making the outer member 312 of a plastic non-conductive material having a conductance of less than about 100 ohms per square inch. Additionlly, and alternatively, the outer sleeve 312 may be covered with an insulated elastomer such as silicone, latex or natural rubber, or the outer sleeve 312 may be coated with a conductive material having a conductance of less than about 100 ohms per square are inch to provide an electrically resistant coating 312A.

When it is desired to insert the trocar assembly 310 into the abdominal wall through an incision, or the like, during surgery, the assembly 310 is inserted therein by the surgeon simply grasping the housing 311 and inserting the assembly 310 through such incision, or opening. Thereafter, prior to introduction of the electrically activated auxiliary instrument I through the assembly 310, the control 333 is contacted by the finger or thumb of the surgeon and moved from the position shown in Fig. 14, backwardly, to the position shown in Fig. 15. Accordingly, as the control 333 is manipulated, the teeth 321 in the ring 321A will travel across the companion inter-engaging teeth 320 in the configuration 322 of the gear member 319, such that the first elongate cylindrical member 312 moves away from the no-go shoulder 327 on the cylindrical extension 326.

Because the first and second cylindrical members 312, 313 and 313A are attached one to another by means of the securements 330 at the distal end of the assembly 310, movement of the first elongate cylindrical member 312 relative to the second member, 313, will cause the sleeve portion 325 flexing members 323 to be urged regularly outwardly from the cylindrical member 313 from the retracted outer diameter of position 317 (Fig. 12) to the expanded outer diameter of 318 (Fig. 13). In the position shown in Fig. 13, the trocar assembly 310 thus resists removal from the abdominal wall W in the cavity, during surgery.

The apparatus may be moved from the position shown in Fig. 13 to the position shown in Fig. 12 by reversing the procedure described above, and the assembly 310 completely withdrawn from the abdominal wall W.

Accordingly, as the electrically activated auxiliary instrument I is introduced within the assembly 310, arcing between the metallic housing of such instrument I and the metal sleeve 313 is prevented by provision of the insert sleeve member 313A which provides insulation means. If such sleeve 313A is not provided, but the outer sleeve 312 is made of a conductive plastic, as described herein, shocking of the patient will be eliminated because such plastic sheath will not act as a capacitor therefore dissipate any electrical shock to the patient during surgery.

## Claims

1. A trocar instrument for use in inserting a laparoscopic instrument into an abdominal cavity, the instrument comprising a tubular trocar sleeve (28,104,313) which is arranged to contain a trocar (12) with a sharp end of the trocar projecting from a first end of the sleeve, whereby, in use, an incision can be made in the abdominal wall and the first end inserted through the incision into the abdominal cavity whereafter the trocar (12) may then be withdrawn through the sleeve (28,313) and replaced by the laparoscopic instrument; a radially expandable portion (20,90,92,323) at the first end of the sleeve; means capable of expanding the portion when the first end has been inserted through the incision whereby the expanded portion abuts the inner abdominal wall about the incision to resist withdrawal of the sleeve from the abdominal cavity; and an insulating means (28A,104A,312A,313A) for insulating an operator or patient from an electrically activated laparoscopic instrument inserted, in use, into the trocar sleeve.

2. An instrument according to claim 1, wherein the insulating means is an insulating sleeve or coating (28A,104A) disposed on the inside of the trocar sleeve (28,104).

3. An instrument according to claim 1, wherein the insulating means is a sleeve or coating (312A) disposed on the outside of the trocar sleeve (313).

4. An instrument according to claim 3, wherein the sleeve or coating (312A) is elastomeric.

5. An instrument according to claim 1, wherein the sleeve comprises a first sleeve (313) surrounded by a second sleeve (312) which is provided with the radially expandable portion, and wherein the means capable of expanding the portion comprise a first gear member (319) on the second sleeve (312) including first interengagable means (320) extending along the second sleeve; and a second gear member (321A) being carried on a housing (311) having second interengagable means (321) for companion interfacing with the first interengagable means and being rotatable about an axis transversely to the axis of the first and second sleeves (312,313), to move the radially expandable portion (20,90,92,323) into an expanded position.

6. An instrument according to claim 1, wherein the trocar sleeve comprises a first sleeve (313) surrounded by a second sleeve (312) which is provided with the radially expandable portion (323); wherein the insulating means is a sleeve or coating (313A) disposed between the first and second sleeves.

7. An instrument according to according to any one of the preceding claims, wherein the insulating means is formed of a conductive plastic having an electrical conductance of less than about 100 ohms per square inch, to dissipate charge from the trocar assembly during the insertion of the instrument.

8. A laparoscopic surgical method, comprising the steps of forming an incision through the abdominal wall of a patient; introducing through the incision a laparoscopic trocar assembly comprising a trocar sleeve (28,104 313) having a first end which, in use, extends into the abdomen through the incision in the abdominal wall, the first end of the sleeve having a first external dimension passage through the incision, a radially expandable portion (20,90,92,323) for expanding a portion of the first end of the sleeve within the abdomen, a passageway through the assembly and interior of the sleeve for introduction and removal of the laparoscopic instrument; insulation means (28A,104A,312A,313A) to electrically insulate the trocar assembly from an operator or the patient while the instrument is within the assembly; and expanding the first end of the sleeve within the abdomen to a larger, second external dimension, the first end of the sleeve abutting the inner abdominal wall about the incision when expanded to the second external dimension to resist the withdrawal of the trocar assembly from the abdominal cavity.

9. A method according to claim 8, further comprising the step of introducing an electrically activated laparoscopic instrument through the passageway and into the abdominal cavity.

10. A method according to claim 9, further comprising the steps of reducing the external dimension of the first end from the second external dimension to the first external dimension while the laparoscopic trocar sleeve is within the abdomen; and withdrawing the electrically activated laparoscopic instrument through the laparoscopic trocar assembly.

11. A method according to claim 9, further comprising the steps of withdrawing the electrically activated laparoscopic instrument from the abdomen out of the trocar assembly, and retracting the portion of the first end of the sleeve (28,104,313) within the abdomen so that the external dimension of the first end is retracted to the first external dimension prior to withdrawal of the assembly from the abdominal cavity.
